# EUROPEAN PATENT SPECIFICATION

(11) **EP 3 570 882 B1**
(45) Date of publication and mention of the grant of the patent: **17.11.2021**
(21) Application number: 18702623.2
(22) Date of filing: 16.01.2018
(51) Int. Cl.: A61K 39/395, C07K 16/36, A61P 7/02

(54) **NOVEL STABLE FORMULATION FOR FXIA ANTIBODIES**
NEUE STABILE FORMULIERUNGEN VON FACTOR XIA ANTIKÖRPERN
DES FORMULES NOUVELLES STABLES POUR DES ANTICORPS FXIA

(30) Priority: 19.01.2017 EP 17152108
(43) Date of publication of application: 27.11.2019
(73) Proprietor: Bayer Pharma Aktiengesellschaft, 13353 Berlin (DE)
(72) Inventor: OLBRICH, Carsten, 10961 Berlin (DE); TRILL, Thomas, 16552 Schildow (DE); VEURINK, Marieke, 22085 Hamburg (DE)
(74) Representative: BIP Patents
(86) International application number: PCT/EP2018/050951
(87) International publication number: WO 2018/134184

(56) References cited:
- WO-A1-2013/167669
- WO-A1-2015/059147
- WO-A2-2004/055164
- NICHOLAS W WARNE ED - LENDLEIN ANDREAS ET AL: "Development of high concentration protein biopharmaceuticals: The use of platform approaches in formulation development", EUROPEAN JOURNAL OF PHARMACEUTICS AND BIOPHARMACEUTICS, ELSEVIER SCIENCE PUBLISHERS B.V., AMSTERDAM, NL, vol. 78, no. 2, 3 March 2011 (2011-03-03), pages 208-212, XP028203394, ISSN: 0939-6411, DOI: 10.1016/J.EJPB.2011.03.004 [retrieved on 2011-03-13]
- SUNDARAMURTHI PRAKASH ET AL: "Calorimetry and complementary techniques to characterize frozen and freeze-dried systems", ADVANCED DRUG DELIVERY REVIEWS, vol. 64, no. 5, 21 December 2011 (2011-12-21), pages 384-395, XP028910125, ISSN: 0169-409X, DOI: 10.1016/J.ADDR.2011.12.004

### SEQUENCE LISTING

<110> Bayer Pharma AG
<120> Neue stabile Formulierung für FXIa Antikörper
<130> BHC161051
<160> 2
<170> PatentIn version 3.5
<210> 1
   <211> 449
   <212> PRT
   <213> Artificial Sequence
<220>
   <223> schwere Kette anti-FXIa Antikörper BAY1213790
<400> 1
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Gln Tyr
               20                  25                  30
   Gly Met Asp Trp Val Arg Gln Ala Pro Gly Lys Gly Leu Glu Trp Val
           35                  40                  45
   Ser Gly Ile Gly Pro Ser Gly Gly Ser Thr Val Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Thr Arg Gly Gly Pro Tyr Tyr Tyr Tyr Gly Met Asp Val Trp Gly Gln
               100                 105                 110
   Gly Thr Thr Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Lys Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly
<210> 2
   <211> 214
   <212> PRT
   <213> Artificial Sequence
<220>
   <223> leichte Kette anti-FXIa Antikörper BAY1213790
<400> 2
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Asp Ala Ser Asn Leu Glu Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Gln Gln Ala Asp Ser Phe Pro Val
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210

## Description

### Introduction

The present invention refers to novel liquid pharmaceutical formulation comprising human antibody against coagulation factor FXIa as active ingredient, especially those described in WO2013167669A1. The invention also refers to lyophilizates of the specified liquid formulation and also to the use thereof in the therapy and prophylaxis of thrombotic or thromboembolic disorders.

Blood coagulation is a protective mechanism of the organism which helps to be able to "seal" defects in the wall of the blood vessels quickly and reliably. Thus, loss of blood can be avoided or kept to a minimum. Haemostasis after injury of the blood vessels is affected mainly by the coagulation system in which an enzymatic cascade of complex reactions of plasma proteins is triggered. Numerous blood coagulation factors are involved in this process, each of which factors converts, on activation, the respectively next inactive precursor into its active form. At the end of the cascade comes the conversion of soluble fibrinogen into insoluble fibrin, resulting in the formation of a blood clot. In blood coagulation, traditionally the intrinsic and the extrinsic system, which end in a final joint reaction path, are distinguished.

Coagulation factor FXIa is a central component of the transition from initiation to amplification and propagation of coagulation: in positive feedback loops, thrombin activates, in addition to factor V and factor VIII, also factor XI to factor XIa, whereby factor IX is converted into factor IXa, and, via the factor IXa/factor VIIIa complex generated in this manner, the factor X is activated and thrombin formation is in turn therefore highly stimulated leading to strong thrombus growth and stabilizing the thrombus. Anti-FXIa antibodies are known in the prior art as anticoagulants, i.e. substances for inhibiting or preventing blood coagulation (see WO2013167669A1). BAY1213790 is an anti-FXIa antibody comprising the sequence of the heavy chain according to SEQ ID NO: 1 and the light chain according to SEQ ID NO: 2.

Therapeutic proteins such as, for example, human monoclonal antibodies are generally administered by injection as liquid pharmaceutical formulations owing to their properties. Since many therapeutically effective human monoclonal antibodies have unfavourable properties such as low stability or a tendency to aggregation, it is necessary to modulate these unfavourable properties by suitable pharmaceutical formulation. An aggregate or denatured antibody may have, for example, a low therapeutic efficacy. An aggregate or denatured antibody may also provoke undesired immunological reactions. Stable pharmaceutical formulations of proteins should also be suitable to prevent chemical instabilities. Chemical instability of proteins may lead to degradation or fragmentation and thus reduced efficacy or even to toxic side effects. The formation or generation of all types of low-molecular-weight fragments should therefore be avoided or at least minimized. These are all factors which may affect the safety of a preparation and therefore must be influenced. Furthermore, a low viscosity is of advantage when using syringes or pumps since this keeps the force required low and therefore increases the injectability. A low viscosity is also advantageous during production, for example, enabling the precise filling of a preparation. The therapeutic use of a human monoclonal antibody, however, often requires the use of high antibody concentration, which often leads to problems with high viscosity. In their overview article, Daugherty and Mrsny (Adv Drug Deliv Rev. 2006;58(5-6):686-706) discuss this 2006;58(5-6):686-706) discuss this and other problems which can occur in the liquid pharmaceutical formulation of monoclonal antibodies.

A liquid formulation should stabilize an antibody for the longest time possible and also enable lyophilization. A suitable liquid pharmaceutical formulation must therefore stabilize both the biological efficacy of the antibody and the biophysical properties of a human monoclonal antibody. There exists a need, therefore, for concentrated liquid formulations of monoclonal antibodies which comprise a low fraction of aggregates and degradation products, are stable over a long period, can be lyophilized and have minimal viscosity.

The present invention addresses the need mentioned above and provides liquid pharmaceutical formulations comprising anti-FXIa antibodies and low amounts of aggregates and degradation products and from which a stable lyophilizate can also be produced. These formulations also have a low viscosity and may therefore be simply administered to patients, for example by means of syringes or autoinjectors.

WO2013167669 describes a lyophilized pharmaceutical composition of anti-FXIa antibody BAY1213790 provided as a lyophilized powder in a histidine buffer system which comprises 0.1%-2% sucrose and 2%-7% mannitol at a pH range of 4.5 to 5.5.

The invention provides stable liquid pharmaceutical formulations comprising anti-FXIa antibody BAY1213790 and a histidine-glycine buffer system at pH 6 which can also be lyophilized.

### Description of the invention

In one embodiment, the liquid pharmaceutical formulation comprises 5-30 mM histidine and 100-200 mM glycine. In a preferred embodiment, the liquid pharmaceutical formulation comprises 5-10 mM histidine and 130-200 mM glycine. In a particularly preferred embodiment, the liquid pharmaceutical formulation comprises 10 mM histidine and 130 mM glycine. Furthermore, the liquid pharmaceutical formulation has a pH of 5.5 **―** 7.5. In a preferred embodiment, the liquid pharmaceutical formulation has a pH of 5.7 **―** 6.3. In a particularly preferred embodiment, the liquid pharmaceutical formulation has a pH of 6. The liquid pharmaceutical formulation according to the invention comprises anti-FXIa antibodies at concentrations of 5 **―** 50 mg/ml. In a preferred embodiment, the anti-FXIa antibody is present at concentrations of 10 **―** 40 mg/ml. In a particularly preferred embodiment, the anti-FXIa antibody has a concentration of 25 mg/ml. In all embodiments, the anti-FXIa antibody is particularly preferably BAY1213790. The liquid pharmaceutical formulation may also comprise a stabilizer. Stabilizers are sugars for example. "Sugars" refers to a group of organic compounds which are water-soluble and are divided among monosaccharides, disaccharides and polyols. A preferred sugar is a non-reducing disaccharide, particular preference being given to sucrose or trehalose dihydrate. In one embodiment, the stabilizer is present to an extent of 1-10% weight to volume (w/v), preferably to an extent of 3-7% (w/v) and particularly preferably to an extent of 5% (w/v). In a preferred embodiment, trehalose dihydrate is present to an extent of 1-10% weight to volume (w/v), preferably to an extent of 3-7% (w/v) and particularly preferably to an extent of 5% (w/v). The liquid pharmaceutical formulation may also comprise a wetting agent. The term "wetting agent" refers to any detergent having a hydrophilic and a hydrophobic region and includes non-ionic, cationic, anionic and zwitterionic detergents. Preferred detergents may be selected from the group consisting of polyoxyethylene sorbitan monooleate (also known as polysorbate 80 or TWEEN 80), polyoxyethylene sorbitan monolaurate (also known as polysorbate 20 or TWEEN 20) and N-laurylsarcosine. For the compositions disclosed, preference is given to a non-ionic wetting agent. Particular preference is given to the use of polysorbate 80 for the compositions of the present invention. The wetting agent may be used at a concentration of 0.001% to 0.5% (w/v), preference being given to a concentration range of 0.005% to 0.1% (w/v). Particular preference is given to using a wetting agent concentration of 0.01% (w/v).

Preservatives or other additives, fillers, stabilizers or carriers may optionally be added to the liquid pharmaceutical formulations according to the invention. Suitable preservatives are, for example, octadecyldimethylbenzylammonium chloride, hexamethonium chloride, and aromatic alcohols such as phenol, parabens or m-cresol. Further pharmaceutically acceptable additives, stabilizers or carriers are described, for example, in Remington's Science And Practice of Pharmacy (22nd edition, Loyd V. Allen, Jr, editor. Philadelphia, PA: Pharmaceutical Press. 2012).

The invention therefore provides a liquid pharmaceutical formulation comprising the anti-FXIa antibody BAY1213790 and a histidine-glycine buffer system, wherein the formulation comprises 5-30 mM histidine and 100-200 mM glycine, preferably 5-10 mM histidine and 130-200 mM glycine and has a pH of 5.5 **―** 6.5, preferably 5.7 **―** 6.3. This produces a sufficient stabilization and low aggregation of the antibody BAY1213790 at low viscosity and also enables optional lyophilization of the formulation.

One embodiment according to the invention is a liquid pharmaceutical formulation comprising
anti-FXIa antibody BAY1213790 at a concentration of 5 ― 50 mg/ml, preferably 10 ― 40 mg/ml,
5-30 mM histidine and 100-200 mM glycine, preferably 5-10 mM histidine and 130-200 mM glycine, more preferably 10 mM histidine and 130 mM glycine,
wherein the formulation has a pH of 5.5 **―** 6.5, preferably 5.7 **―** 6.3, more preferably 6. The formulation optionally comprises further ingredients selected from the group consisting of wetting agents, preservatives, carriers and stabilizers.

One embodiment according to the invention is a liquid pharmaceutical formulation comprising
anti-FXIa antibody BAY1213790 at a concentration of 5 **―** 50 mg/ml, preferably 10 **―** 40 mg/ml,
5-30 mM histidine and 100-200 mM glycine, preferably 5-10 mM histidine and 130-200 mM glycine, more preferably 10 mM histidine and 130 mM glycine,
1 ― 10% (w/v) stabilizer, preferably 3 ― 7% (w/v) trehalose dihydrate,
wherein the formulation has a pH of 5.5 ― 6.5, preferably 5.7 ― 6.3, more preferably 6. The formulation optionally comprises further ingredients selected from the group consisting of wetting agents, preservatives, carriers and stabilizers.

A preferred embodiment is a liquid pharmaceutical formulation comprising
anti-FXIa antibody BAY1213790 at a concentration of 10 ― 40 mg/ml,
5-10 mM histidine and 130-200 mM glycine,
3 ― 7% (w/v) trehalose dihydrate, and
polysorbate 80 at a concentration of 0.005% to 0.1% (w/v),
wherein the formulation has a pH of 5.7 - 6.3. The formulation optionally comprises further ingredients selected from the group consisting of preservatives, carriers and stabilizers.

A particularly preferred embodiment is a liquid pharmaceutical formulation comprising
anti-FXIa antibody BAY1213790 at a concentration of 25 mg/ml,
10 mM histidine and 130 mM glycine,
5% (w/v) trehalose dihydrate, and
polysorbate 80 at a concentration of 0.05% (w/v),
wherein the formulation has a pH of 6. The formulation optionally comprises further ingredients selected from the group consisting of preservatives, carriers and stabilizers.

The term "buffer" describes herein a buffered solution, the pH of which changes only slightly after addition of acidic or alkaline substances. Buffered solutions contain a mixture of a weak acid and its corresponding base or of a weak base and its corresponding acid.

The term "patient" refers to human or animal individuals receiving a preventive or therapeutic treatment.

The term "treatment" herein refers to the use or administration of a therapeutic substance on/to a patient, or to the use or administration of a therapeutic substance on/to an isolated tissue or on/to a cell line of a patient, who is suffering from a disease, is showing a symptom of a disease, or has a predisposition to a disease, with the goal of curing, improving, influencing, stopping or alleviating the disease, its symptoms or the predisposition to the disease.

"Effective dose" describes herein the active-ingredient amount with which the desired effect can be at least partially achieved. A "therapeutically effective dose" is therefore defined as the active-ingredient amount which is sufficient to at least partially cure a disease, or to at least partially eliminate adverse effects in the patient that are caused by the disease. The amounts actually required for this purpose are dependent on the severity of the disease and on the general immune status of the patient.

An "isotonic solution" has substantially the same osmotic pressure as human blood. Isotonic solutions therefore have in general an osmotic pressure of about 250 to 350 mOsm. The term "hypotonic" describes compositions having an osmotic pressure below that of human blood, whereas "hypertonic" compositions have an osmotic pressure above that of human blood.

The term "high-molecular-weight aggregates" (synonym: "HMW") describes aggregates which are composed of at least two protein monomers.

The invention further provides a product which comprises one of the pharmaceutical formulations according to the invention and preferably also instructions for use. In one embodiment, the product comprises a container which comprises liquid formulations or lyophilizates according to the invention. Useable containers are, for example, bottles, vials, tubes or syringes. The containers can, for example, be composed of glass or plastic. Syringes can comprise an injection needle composed, for example, of metal. The invention further provides a kit which comprises the aforementioned pharmaceutical formulations.

In one embodiment, the container is a syringe. In a further embodiment, the syringe is contained in an injection device. Preference is given to an administration via an intravenous (rapid) infusion after dilution with standard infusion solutions such as 0.9% NaCl solution.

The compositions according to the invention exhibit increased stability compared to the formulations for anti-FXIa antibodies available in the prior art. The preferred formulations are suitable as liquid formulations but for more stringent requirements can also be lyophilized. The liquid pharmaceutical formulation according to the invention accordingly may also be a reconstituted lyophilizate. Owing to this property profile, the liquid pharmaceutical formulations according to the invention are especially suitable for parenteral administration. Parenteral administrations includes, inter alia, intravenous injection or infusion, intra-arterial injection or infusion (into an artery), intra-muscular injection, intra-thecal injection, subcutaneous injection, intra-peritoneal injection or infusion, intra-osseous administration or injection into a tissue. The compositions according to the invention are especially suitable for intravenous or subcutaneous administration.

The liquid pharmaceutical formulations according to the invention exhibit high stability in long term tests, even as a lyophilizate. They also exhibit an excellent reconstitution while maintaining the biological activity. The liquid pharmaceutical formulations according to the invention may also be freeze-dried such that they comprise at most 2% residual moisture. A further embodiment of the invention is accordingly a lyophilizate obtainable by freeze-drying of a liquid formulation according to the invention. Preference is given to a lyophilizate comprising at most 2% residual water. Particular preference is given to a lyophilizate comprising at most 1% residual water.

The liquid pharmaceutical formulations according to the invention have valuable pharmacological properties and can be used for prevention and treatment of diseases in humans and animals. The liquid pharmaceutical formulations according to the invention which may be employed for diseases and treatment thereof particularly include the group of thrombotic or thromboembolic diseases. Accordingly, the liquid pharmaceutical formulations according to the invention are suitable for the treatment and/or prophylaxis of diseases or complications which may arise from the formation of clots.

In the context of the present invention, the "thrombotic or thromboembolic diseases" include diseases which occur both in the arterial and in the venous vasculature and which can be treated with the liquid pharmaceutical formulations according to the invention, in particular diseases in the coronary arteries of the heart, such as acute coronary syndrome (ACS), myocardial infarction with ST segment elevation (STEMI) and without ST segment elevation (non-STEMI), stable angina pectoris, unstable angina pectoris, reocclusions and restenoses after coronary interventions such as angioplasty, stent implantation or aortocoronary bypass, but also thrombotic or thromboembolic diseases in further vessels leading to peripheral arterial occlusive disorders, pulmonary embolisms, venous thromboembolisms, venous thromboses, in particular in deep leg veins and kidney veins, transitory ischaemic attacks and also thrombotic stroke and thromboembolic stroke.

Stimulation of the coagulation system may occur by various causes or associated disorders. In the context of surgical interventions, immobility, confinement to bed, infections, inflammation or cancer or cancer therapy, inter alia, the coagulation system can be highly activated, and there may be thrombotic complications, in particular venous thromboses. The liquid pharmaceutical formulations according to the invention are therefore suitable for the prophylaxis of thromboses in the context of surgical interventions in patients suffering from cancer. The liquid pharmaceutical formulations according to the invention are therefore also suitable for the prophylaxis of thromboses in patients having an activated coagulation system, for example in the stimulation situations described.

The liquid pharmaceutical formulations according to the invention are therefore also suitable for the prevention and treatment of cardiogenic thromboembolisms, for example brain ischaemias, stroke and systemic thromboembolisms and ischaemias, in patients with acute, intermittent or persistent cardiac arrhythmias, for example atrial fibrillation, and in patients undergoing cardioversion, and also in patients with heart valve disorders or with artificial heart valves.

In addition, the liquid pharmaceutical formulations according to the invention are suitable for the treatment and prevention of disseminated intravascular coagulation (DIC) which may occur in connection with sepsis inter alia, but also owing to surgical interventions, neoplastic disorders, burns or other injuries and may lead to severe organ damage through microthromboses.

Thromboembolic complications furthermore occur in microangiopathic haemolytical anaemias and by the blood coming into contact with foreign surfaces in the context of extracorporeal circulation, for example haemodialysis, ECMO ("extracorporeal membrane oxygenation"), LVAD ("left ventricular assist device") and similar methods, AV fistulas, vascular and heart valve prostheses.

Moreover, the liquid pharmaceutical formulations according to the invention are suitable for the treatment and/or prophylaxis of diseases involving microdot formations or fibrin deposits in cerebral blood vessels which may lead to dementia disorders such as vascular dementia or Alzheimer's disease. Here, the clot may contribute to the disorder both via occlusions and by binding further disease-relevant factors.

Moreover, the liquid pharmaceutical formulations according to the invention can be used for inhibiting tumour growth and the formation of metastases, and also for the prophylaxis and/or treatment of thromboembolic complications, for example venous thromboembolisms, for tumour patients, in particular those undergoing major surgical interventions or chemo- or radiotherapy.

In addition, the liquid pharmaceutical formulations according to the invention are also suitable for the prophylaxis and/or treatment of pulmonary hypertension.

In the context of the present invention, the term "pulmonary hypertension" includes pulmonary arterial hypertension, pulmonary hypertension associated with disorders of the left heart, pulmonary hypertension associated with pulmonary disorders and/or hypoxia and pulmonary hypertension owing to chronic thromboembolisms (CTEPH).

"Pulmonary arterial hypertension" includes idiopathic pulmonary arterial hypertension (IPAH, formerly also referred to as primary pulmonary hypertension), familial pulmonary arterial hypertension (FPAH) and associated pulmonary arterial hypertension (APAH), which is associated with collagenoses, congenital systemic-pulmonary shunt vitia, portal hypertension, HIV infections, the ingestion of certain drugs and medicaments, with other disorders (thyroid disorders, glycogen storage disorders, Morbus Gaucher, hereditary teleangiectasia, haemoglobinopathies, myeloproliferative disorders, splenectomy), with disorders having a significant venous/capillary contribution, such as pulmonary-venoocclusive disorder and pulmonary-capillary haemangiomatosis, and also persisting pulmonary hypertension of neonatants.

Pulmonary hypertension associated with disorders of the left heart includes a diseased left atrium or ventricle and mitral or aorta valve defects.

Pulmonary hypertension associated with pulmonary disorders and/or hypoxia includes chronic obstructive pulmonary disorders, interstitial pulmonary disorder, sleep apnoea syndrome, alveolar hypoventilation, chronic high-altitude sickness and inherent defects.

Pulmonary hypertension owing to chronic thromboembolisms (CTEPH) comprises the thromboembolic occlusion of proximal pulmonary arteries, the thromboembolic occlusion of distal pulmonary arteries and non-thrombotic pulmonary embolisms (tumour, parasites, foreign bodies).

The present invention further provides for the use of the liquid pharmaceutical formulations according to the invention for production of medicaments for the treatment and/or prophylaxis of pulmonary hypertension associated with sarcoidosis, histiocytosis X and lymphangiomatosis.

In addition, the liquid pharmaceutical formulations according to the invention are also suitable for the treatment and/or prophylaxis of disseminated intravascular coagulation in the context of an infectious disease, and/or of systemic inflammatory syndrome (SIRS), septic organ dysfunction, septic organ failure and multiorgan failure, acute respiratory distress syndrome (ARDS), acute lung injury (ALI), septic shock and/or septic organ failure.

In the course of an infection, there may be a generalized activation of the coagulation system (disseminated intravascular coagulation or consumption coagulopathy, hereinbelow referred to as "DIC") with microthrombosis in various organs and secondary haemorrhagic complications. Moreover, there may be endothelial damage with increased permeability of the vessels and diffusion of fluid and proteins into the extravasal space. As the infection progresses, there may be failure of an organ (for example kidney failure, liver failure, respiratory failure, central-nervous deficits and cardiovascular failure) or multiorgan failure.

In the case of DIC, there is a massive activation of the coagulation system at the surface of damaged endothelial cells, the surfaces of foreign bodies or crosslinked extravascular tissue. As a consequence, there is coagulation in small vessels of various organs with hypoxia and subsequent organ dysfunction. A secondary effect is the consumption of coagulation factors (for example factor X, prothrombin and fibrinogen) and platelets, which reduces the coagulability of the blood and may result in heavy bleeding.

The liquid pharmaceutical formulations according to the invention are also suitable for the primary prophylaxis of thrombotic or thromboembolic disorders and/or inflammatory disorders and/or disorders with increased vascular permeability in patients in which gene mutations lead to enhanced activity of the enzymes, or increased levels of the zymogens and these are established by relevant tests/measurements of the enzyme activity or zymogen concentrations.

The present invention further provides for the use of the liquid pharmaceutical formulations according to the invention for the treatment and/or prophylaxis of disorders, especially the disorders mentioned above.

The present invention further provides for the use of the liquid pharmaceutical formulations according to the invention for production of a medicament for the treatment and/or prophylaxis of disorders, especially the disorders mentioned above.

The present invention further provides a method for treatment and/or prophylaxis of disorders, especially the disorders mentioned above, using a therapeutically effective amount of an inventive compound.

The present invention further provides the liquid pharmaceutical formulations according to the invention for use in a method for the treatment and/or prophylaxis of disorders, especially the disorders mentioned above, using a therapeutically effective amount of a compound according to the invention.

These well described diseases in humans can also occur with a comparable aetiology in other mammals and can be treated there with the liquid pharmaceutical formulations of the present invention.

In the context of this invention, the term "treatment" or "treat" is used in the conventional sense and means attending to, caring for and nursing a patient with the aim of combating, reducing, attenuating or alleviating a disease or health abnormality, and improving the living conditions impaired by this disease.

The present invention therefore further provides for the use of the liquid pharmaceutical formulations according to the invention for the treatment and/or prevention of disorders, especially the disorders mentioned above.

The present invention further provides for the use of the liquid pharmaceutical formulations according to the invention for production of a medicament for the treatment and/or prevention of disorders, especially the disorders mentioned above.

The present invention further provides for the use of the liquid pharmaceutical formulations according to the invention in a method for treatment and/or prevention of disorders, especially of the aforementioned disorders.

The present invention further provides a method for treating and/or preventing diseases, more particularly the aforementioned diseases, using an effective amount of one of the liquid pharmaceutical formulations according to the invention.

In a preferred embodiment, the treatment and/or prevention is parenteral administration of the liquid pharmaceutical formulations according to the invention. Particular preference is given to intravenous or subcutaneous administration.

The pharmaceutical formulations according to the invention can be used alone or, if required, in combination with one or more other pharmacologically active substances, provided that this combination does not lead to undesirable and unacceptable side effects. The present invention therefore further provides medicaments comprising at least one of the compositions according to the invention and one or more further active ingredients, especially for the treatment and/or prevention of the aforementioned diseases.

The liquids according to the invention can be administered as a single treatment but can also be administered repeatedly successively, or can be administered long-term following diagnosis.

### Examples

### Description of the analytical methods

### Protein concentration (UV/VIS spectroscopy):

The protein concentration is determined by absorption at 280 nm. For possible light scattering, the test is also corrected at 320 nm.

### Buffer screening:

Formulation buffer tests follow the principle which follows:
Thermal stability of the antibody (unfolding) is determined by means of a temperature profile (T: 15 to 105°C) by a DSC method (DSC: Differential Scanning Calorimetry). The so-called thermal unfolding (TM₁) is a measure for comparing various buffer systems: With increasing TM₁ values, the thermal stability of the protein increases. A "higher" TM₁ is therefore an indication of good stability of the antibody in the relevant buffer system.

### Visual image:

The samples are assessed visually by protected personnel for the presence of visible particles and their appearance (flakes/fibres) (visual check). Solutions should be as free of visible particles as possible.

### Protein recovery (C₂₈₀):

During rebuffering on the Äkta (chromatography), it is possible to determine a recovery rate, since the antibody can accumulate on various surfaces during the process. A high recovery rate during/after completion of the process is therefore an important prerequisite for a successful end product.

### pH measurement:

Measurements are always conducted at the same temperature (20-25°C). The instrument is calibrated with 2 standard solutions every day after which a control is measured which must not deviate by more than 0.05 pH units.

SEC (HMW, monomer, LMW) in combination with UV detector (280nm):
SEC (size exclusion chromatography) separates monomers from fragments (low molecular weight LMW) and oligomers (high molecular weight HMW) based on their spatial size. The monomer fraction should be as high as possible here.

### DLS (median value):

A further quality criterion of pharmaceutical antibody solutions can be determined by dynamic light scattering (DLS). Here, the light scattering of the molecules is used to determine their hydrodynamic radius. The hydrodynamic radius of an antibody is dependent on its conformation, inter alia. During the stress test, the conformation of the antibody can change due to unfolding or self-association - the change is detectable by DLS
1) With increasing concentration of the antibody in solution, the intermolecular interactions increase. If the forces have an attractive effect here, the antibodies tend to so-called oligomer formation, i.e. the individual monomers formerly present now form entities of several antibodies. The measured hydrodynamic radius increases since the antibody entities appear "larger".
2) It is possible that the surrounding medium has a favourable/unfavourable charge for the antibody; in this case it can result in individual monomers denaturing (unfolding); the measured hydrodynamic radius would thus likewise appear "enlarged".

When considering the DLS results, it should be taken into account that during development two different instruments were used for the DLS measurements. In Berlin, the LB-550 from HORIBA Instruments was used. In Wuppertal, the Zetasizer Nano-ZS from Malvern was used. Differences in composition and in the sample measurement may lead to slightly different values. For this reason, the results should not be compared! However, the trend of the values with both instruments could be reproduced. Each table below describes with which instrument the values obtained have been measured.

### A2 value:

In the measurement of the second virial coefficient (A2 value), the development of the molecular weight is recorded by static light scattering. In this case, intermolecular interactions can be monitored, similar to the measurement of the dynamic light scattering. If the molecular masses increase super-proportionately with increasing concentration, the antibodies tend to aggregation - the predominant conditions in the formulation are referred to as "attractive". If, in contrast, the molecular masses develop sub-proportionately, "repulsive" conditions prevail in the system. The tendency to aggregation is limited.

### CGE:

Capillary electrophoresis is an analytical method for separating molecules in an electrical field due to their charge. Shape and size of the molecules also play a role in the separation by a gel-like medium, so that here also - similar to size exclusion chromatography, the antibody and fragments or aggregates thereof are separated.

### Cloudiness:

The cloudiness of the solutions is carried out with the aid of a turbidity measurement. In this case, a light of defined luminosity is passed through the solution and how much energy that is incorporated on the opposite side of the solution can then be detected and recorded. If the cloudiness increases, the turbidity likewise increases - more light is "detained" by the solution.

### Bioassay:

The biochemical test for BAY 1213790 (anti-hFXIa monoclonal antibody) determines the inhibitory activity of the antibody on activated human coagulation factor XI (hFXIa). In this case, the functional neutralization of human FXIa by BAY1213790 is determined using a fluorogenic enzyme activity assay. The EC50 value of the test sample is compared with BAY 1213790 reference standard. This standard has been produced by a comparable production process and is stored in 10 mM histidine/130 mM glycine buffer at pH 6 at <-60°C.

### Biacore:

The binding of the BAY 1213790 test sample and of the BAY 1213790 reference standard to human factor XIa (FXIa) antigen is determined by surface plasmon resonance spectroscopy (SPR, Biacore). The binding affinity of the test sample is compared to the BAY 1213790 reference standard. This standard has been produced by a comparable production process and is stored in 10 mM histidine/130 mM glycine buffer at pH 6 at <-60°C.

### Results:

For the buffer screening, the pH range of the buffer systems which is suitable for parenteral dosage forms was selected in the pH range 5.0 to 7.5. The antibody concentration of the formulations prepared, in the first tests (thermal stability determination), is ca. 1 mg/ml. No further auxiliaries were added.

**Tab. 1: TM₁ (DSC method) of BAY1213790 (1 mg/ml) in various buffer systems**

| **pH** | **5.0** | **5.5** | **6.0** | **6.5** | **7.0** | **7.5** |
|---|---|---|---|---|---|---|
| **PBS (Sigma P3813)** | | | | | | 72.5 |
| **50 mM Na₂HPO₄ x 2H₂O** | 67.0 | 68.6 | 71.7 | 72.1 | 71.8 | 76.0 |
| **50 mM L-histidine** | | 66.3 | 69.2 | 70.7 | 72.5 | 73.1 |
| **50 mM Na acetate** | 69.8 | 66.4 | | | | |
| **50 mM Tris** | | | | | | 71.4 |
| **50 mM Na citrate** | 65.4 | 67.9 | 69.5 | 70.9 | | |
| **50 mM L-arginine** | | | 70.6 | 69.9 | 70.6 | 71.0 |
| **50 mM L-glycine** | | | 72.8 | 78.7 | 73.5 | 78.4 |
| **50 mM L-lysine** | | | 70.0 | 69.9 | 70.2 | 70.9 |
| **10 mM His, 130 mM Gly** | | | 72.0 | 72.3 | 72.5 | 73.6 |

The TM₁ values (unfolding temperature) were between 65.4°C and 78.7°C. Formulations having a TM₁ above 72.0°C were selected for further tests, since protein formulations having a high TM₁ value are an indication of a stabilizing formulation.

Anti-factor XIa Ab BAY1213790 formulations with Na**₂**HPO₄ (pH 6.0 to 7.5), L-glycine (pH 6.0 to 7.5), L-histidine (pH 7.0 to 7.5) and the combination of 10 mM histidine and 130 mM glycine (pH 6.0 to 7.5) showed the highest thermal stabilities.

Since the target concentration of the formulation was established at 25 mg/mL, the protein was rebuffered into the selected buffer systems in a next step using a preparative SEC system and concentrated using Vivapore 10/20 (from ca. 6 to 40 mg/ml).

**Tab.2: Rebuffering/concentration of BAY1213790**

| **Buffer system** | **C_{Protein} [mg/ ml]¹** | **Recovery [%]** | **SEC HMW [%]** | **SEC Monomer [%]** | **SEC LMW [%]** | **Visuall y** |
|---|---|---|---|---|---|---|
| **50 mM Na₂HPO₄ x 2H₂O pH 6.0** | 37.63 | 93.1 | 1.28 | 98.7 | 0.03 | ok |
| **50 mM Na₂HPO₄ x 2H₂O pH 6.5** | 43.48 | 106.8 | 1.46 | 98.5 | 0.04 | ok |
| **50 mM Na₂HPO₄ x 2H₂O pH 7.0** | 43.12 | 103.7 | 1.62 | 98.3 | 0.03 | ok |
| **50 mM Na₂HPO₄ x 2H₂O pH 7.5** | 37.24 | 91.1 | | | | |
| **50 mM L-histidine pH 6.0** | 38.38 | 95.4 | 1.18 | 97.9 | 0.96 | ok |
| **50 mM L-histidine pH 6.5** | 37.98 | 92.1 | - | - | - | ok |
| **50 mM L-histidine pH 7.0** | 33.86 | 83.5 | - | - | - | ok |
| **50 mM L-histidine pH 7.5** | 33.97 | 83.7 | - | - | - | ok |
| **10 mM L-histidine, 130 mM L-glycine pH 6.0** | 39.31 | 97.8 | 1.01 | 98.7 | 0.29 | ok |
| **10 mM L-histidine, 130 mM L-glycine pH 6.5** | 34.47 | 84.5 | - | - | - | ok |
| **10 mM L-histidine, 130 mM L-glycine pH 7.0** | 30.03 | 74.6 | - | - | - | ok |
| **10 mM L-histidine, 130 mM L-glycine pH 7.5** | 24.09 | 57.7 | - | - | - | ok |

| | | | | | | |
|---|---|---|---|---|---|---|
| ¹ Owing to different buffer compositions and the effects linked thereto, the samples after rebuffering have different concentrations | | | | | | |

The histidine and histidine/glycine formulations exhibited a pH-dependent protein recovery with the recovery decreasing with increasing pH. Good recovery (≥ 95%) was found at pH 6.0. The phosphate buffers did not show this trend. For the samples with high recovery, SEC was additionally determined. No differences in the monomer content or in the visual image were shown.

Generally, proteins are sensitive to agitation stress, Vigorous shaking may potentially lead to aggregation of the protein and may form oligomers (HMW) up to visible particles. It is known that additions of surfactants such as polysorbate 80 and polysorbate 20 have a protective effect on proteins against surface stress. Polysorbate 80 was added to the selected formulations so that the formulations contained in total 0.01% polysorbate 80.

The stabilities of the anti-factor XIa Ab formulations were tested with the agitation stress test (24 h at 300 rpm and RT). Subsequently, the formulations were investigated for changes by means of the following methods:
1) visual image
2) protein recovery (C₂₈₀):
3) SEC (HMW, monomer, LMW)
4) DLS (median value)

After the agitation stress, all Na₂HPO₄ formulations showed increased oligomers (HMW >2%) in the SEC. Some samples additionally had visible particles.

The histidine formulations at pH 6.5 to 7.5 flocculated during the agitation. The solution at pH 6.0 looked good at the start but the oligomers had increased (2.9%) and the recovery was only 91%.

This shows a serious difference between the samples depending on the buffer system selected.

The histidine/glycine formulation at pH 6.0 showed a good monomer content of 98.0% and the recovery was 95%.

The DLS values (hydrodynamic diameter) of the sodium hydrogen phosphate systems was between d(H): 23 to 30 nm, whereas the diameter of the antibodies in the preferred formulation remained at 19 nm. This difference in the conformation is an indication of the formation of relatively large aggregates (caused by attractive intermolecular interactions) which is confirmed in the analytical investigation by SEC (increase in the HMW) and by visible particles in some solutions.

These observations show that the histidine/glycine system at pH 6.0 has a better stabilizing effect on the BAY 1213790 antibody than other systems although this was not apparent by consideration of the unstressed samples.

**Tab.3: BAY 1213790 in formulations with 0.01% (w/v) polysorbate 80 after agitation stress test**

| **Buffer system** | **C_{Protein} [mg/ ml]¹** | **Recovery [%]** | **SEC HMW [%]** | **SEC Monome r[%]** | **SEC LMW [%]** | **DLS [nm]** | **Visu ally** |
|---|---|---|---|---|---|---|---|
| **50 mM Na₂HPO₄ pH 6.0** | 36.51 | 97.0 | 2.61 | 97.4 | 0.03 | 30 | Parti cles |
| **50 mM Na₂HPO₄ pH 6.5** | 44.72 | 102.9 | 3.10 | 96.8 | 0.05 | 28 | ok |
| **50 mM Na₂HPO₄ pH 7.0** | 39.78 | 92.3 | 5.99 | 94.0 | 0.03 | 25 | Parti cles |
| **50 mM Na₂HPO₄ pH 7.5** | 41.19 | 110.6 | 7.50 | 91.8 | 0.04 | 23 | ok |
| **50 mM histidine pH 6.0** | 34.79 | 90.6 | 2.90 | 96.3 | 0.72 | 22 | ok |
| **50 mM histidine pH 6.5** | 39.72 | 104.6 | - | - | - | - | turbi d |
| **50 mM histidine pH 7.0** | 30.77 | 90.9 | - | - | - | - | flake s |
| **50 mM histidine pH 7.5** | 21.76 | 64.1 | - | - | - | - | flake s |
| **10 mM histidine**, | 37.32 | 94.9 | 1.68 | 98.0 | 0.35 | 19 | ok |
| **130 mM glycine pH 6.0** | | | | | | | |
| **10 mM histidine, 130 mM glycine pH 6.5** | 31.40 | 91.1 | - | - | - | - | flake s |
| **10 mM histidine, 130 mM glycine pH 7.0** | 28.27 | 94.2 | - | - | - | - | flake s |
| **10 mM histidine, 130 mM glycine pH 7.5** | 20.37 | 84.6 | - | - | - | - | flake s |

| | | | | | | | |
|---|---|---|---|---|---|---|---|
| Owing to different buffer compositions and the effects linked thereto, the samples after rebuffering have different concentrations | | | | | | | |

In order to be able to display the effect of various auxiliary concentrations on the antibodies, further tests were carried out in this area. The technology for the determination of the DLS data was modified in the meantime and the tests were also carried out at another concentration (25 mg/mL) such that the results differ slightly but are similar in their trend.

**Tab.4: T0: unstressed samples of BAY 1213790 (25mg/mL) in various buffer systems, all comprising: 0.01% (w/v) polysorbate 80 and at pH 6.0 (DLS values were measured using the Zetasizer Nano-ZS from Malvern)**

| **Sample** | **DLS d(H) [nm]** | **Visually** | **C Protein [mg/ml]** |
|---|---|---|---|
| **5 mM histidine, 200 mM glycine** | 11.12 | ok | 25.38 mg/mL |
| **10 mM histidine, 130 mM glycine** | 13.85 | ok | 24.88 mg/mL |
| **30 mM histidine, 100 mM glycine** | 16.18 | ok | 26.40 mg/mL |
| **50 mM histidine, 50 mM glycine** | 16.97 | ok | 25.77 mg/mL |
| **50 mM Na₂HPO₄** | 18.36 | ok | 25.29 mg/mL |

**Tab.5: T24 (24 h, 300 rpm): stressed BAY 1213790 sample (25 mg/mL) in various buffer systems, all comprising: 0.01% (w/v) polysorbate 80 and at pH 6 (DLS values were measured using the Zetasizer Nano-ZS from Malvern)**

| **Sample** | **DLS d(H) [nm]** | **Visually** | **C Protein [mg/ml]** |
|---|---|---|---|
| **5 mM histidine, 200 mM glycine** | 11.52 | ok | 25.04 mg/mL |
| **10 mM histidine, 130 mM glycine** | 13.72 | ok | 24.92 mg/mL |
| **30 mM histidine, 100 mM glycine** | 16.23 | ok | 26.23 mg/mL |
| **50 mM histidine, 50 mM glycine** | 16.93 | ok | 25.84 mg/mL |
| **50 mM Na₂HPO₄** | 18.21 | Particles | 25.04 mg/mL |

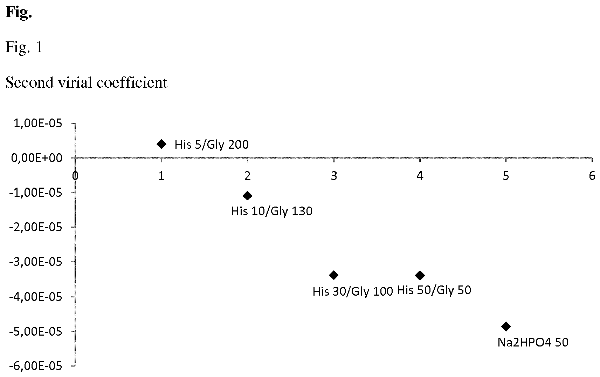

It can be seen that the DLS data of the sodium hydrogen phosphate samples (sample 5) had increased compared to the preferred formulation (sample 2). This phenomenon is due to increased intermolecular interactions which are confirmed by determination of the second virial coefficient (A2 value). In this case, a positive A2 value describes "repulsive" interactions within the system. The antibodies mutually repel due to their surface charge. Negative values describe attractive conditions within the system - which means here that the further the numerical value is from "0", the more pronounced is the effect. In Fig. 1 the measured second virial coefficients of the five unstressed formulations from Table 4 are to be found. It can be seen that sample 1 (5 mM histidine/200 mM glycine at pH 6.0) is the only sample having a positive A2 value and therefore having repulsive interactions. On account of the low buffering capacity of the low histidine proportion, formulation 2 (10 mM histidine/130 mM glycine at pH 6.0) was however selected, which has the lowest negative A2 value in this series, and is nonetheless capable of keeping the pH stable in the formulation during processing and storage.

In the following, the formulations were rendered isotonic with the stabilizers trehalose dihydrate or sucrose.

The four formulations (His/Gly 10/130 mM or 50 mM Na₂HPO₄ each with sucrose or trehalose dihydrate and 0.05% (w/v) polysorbate 80) were subjected to three stress tests:
1. Agitation stress test (24 h at 300 rpm and RT)
2. FTC -80°C (freeze-thaw cycle at -80°C):
   3x freezing and thawing (>2h) at room temperature (>2h)
3. FTC -20°C (freeze-thaw cycle at -20°C):
   3x freezing and thawing (>2h) at room temperature (>2h)

Subsequently, the formulations have been investigated for changes to the stability of BAY 1213790 which included a visual image, protein recovery (C_{Protein}), SEC (HMW, monomer, LMW), CGE non-reduced (1H1L, 2H, 2H1L and IgG) and DLS (median value).

**Tab. 6a: Formulations after preparation and after agitation stress**

| | | | | | | | | | | |
|---|---|---|---|---|---|---|---|---|---|---|
| ***His*/*****Gly:** 10 mM histidine, 130 mM glycine, 0.05% (w*/*v) polysorbate 80, pH 6 and either 5% (w*/*v) trehalose dihydrate or 5% (w*/*v) sucrose* | | | | | | | | | | |
| ***PO₄**: 50 mM Na₂HPO₄, 0.05% (w*/*v) polysorbate 80, pH 6 and either 5% (w*/*v) trehalose dihydrate or 5% (w*/*v) sucrose* | | | | | | | | | | |
| *All formulations comprised 25 mg*/*ml BAY1213790* | | | | | | | | | | |
| *For simplification, the following abbreviations for the excipients are used in the table below: Tre = trehalose dihydrate; Suc = sucrose; Mono = monomer; Vis* = *visual test; CGE analyses: 2H = fragments composed of two heavy chains; 2H1L* = *fragments consisting of two heavy chains and one light chain. (DLS values were measured with the LB-550 from Horiba)* | | | | | | | | | | |

| **Sample** | **C_{Protein} [%]** | **HMW [%]** | **Mono [%]** | **LMW [%]** | **1H1L [%]** | **2H [%]** | **2H1L [%]** | **IgG [%]** | **DLS [nm]** | **Vis** |
|---|---|---|---|---|---|---|---|---|---|---|
| | **After production** | | | | | | | | | |
| **His/Gly, Tre** | - | 1.05 | 97.92 | 1.03 | 0.18 | 0.42 | 2.39 | 96.1 8 | 24 | ok |
| **His/Gly, Suc** | - | 1.03 | 97.92 | 1.05 | 0.18 | 0.37 | 2.50 | 96.1 3 | 21 | ok |
| **PO₄**, **Tre** | - | 1.26 | 97.58 | 1.12 | 0.18 | 0.43 | 2.59 | 95.9 1 | 27 | ok |
| **PO₄**, **Suc** | - | 1.29 | 97.63 | 1.08 | 0.19 | 0.41 | 2.72 | 95.8 1 | 28 | ok |

| | **Afte**r **agitation stress** | | | | | | | | | |
|---|---|---|---|---|---|---|---|---|---|---|
| **His/Gly, Tre** | 101.9 | 0.99 | 98.01 | 1.00 | 0.13 | 0.30 | 2.49 | 96.5 1 | 25 | ok |
| **His/Gly, Suc** | 102.2 | 0.93 | 98.11 | 0.96 | 0.17 | 0.28 | 2.99 | 95.7 3 | 24 | ok |
| **PO₄**, **Tre** | 100.6 | 1.27 | 97.68 | 1.05 | 0.17 | 0.31 | 2.95 | 95.7 4 | 29 | ok |
| **PO₄**, **Suc** | 99.2 | 1.30 | 97.71 | 0.99 | 0.17 | 0.34 | 3.13 | 95.5 2 | 30 | ok |

**Tab. 6b. Formulations after FTC**

| | | | | | | | | | | |
|---|---|---|---|---|---|---|---|---|---|---|
| ***His*/*****Gly:** 10 mM histidine, 130 mM glycine, 0.05% (w*/*v) polysorbate 80, pH 6 and either 5% (w*/*v) trehalose dihydrate or 5% (w*/*v) sucrose* | | | | | | | | | | |
| ***PO₄:** 50 mM Na₂HPO₄, 0.05% (w*/*v) polysorbate 80, pH 6 and either 5% (w*/*v) trehalose dihydrate or 5% (w*/*v) sucrose* | | | | | | | | | | |
| *All formulations comprised 25 mg*/*ml BAY1213790* | | | | | | | | | | |
| *For simplification, the following abbreviations for the excipients are used in the table below: Tre = trehalose dihydrate; Suc = sucrose; Mono = monomer; Vis = visual test; CGE analyses: 2H = fragments composed of two heavy chains; 2H1L = fragments consisting of two heavy chains and one light chain. (DLS values were measured with the LB-550 from Horiba)* | | | | | | | | | | |

| **Sample** | **C_{Protein} [%]** | **HMW [%]** | **Mono [%]** | **LMW [%]** | **1H1L [%]** | **2H [%]** | **2H1L [%]** | **IgG [%]** | **DLS [nm]** | **Vis** |
|---|---|---|---|---|---|---|---|---|---|---|
| | After FTC -80°C | | | | | | | | | |
| **His/Gly, Tre** | 100.4 | 0.97 | 97.67 | 1.36 | 0.16 | 0.31 | 2.56 | 96.1 9 | 26 | ok |
| **His/Gly, Suc** | 102.2 | 0.98 | 97.95 | 1.08 | 0.20 | 0.33 | 2.78 | 96.4 8 | 25 | ok |
| **PO₄**, **Tre** | 100.2 | 1.26 | 97.71 | 1.03 | 0.19 | 0.36 | 2.89 | 95.6 7 | 29 | ok |
| **PO₄**, **Suc** | 99.9 | 1.28 | 97.73 | 0.99 | 0.19 | 0.29 | 2.97 | 95.6 9 | 28 | ok |

| | **After FTC -20°C** | | | | | | | | | |
|---|---|---|---|---|---|---|---|---|---|---|
| **His/Gly, Tre** | 100.7 | 0.98 | 97.94 | 1.08 | 0.22 | - | 2.37 | 96.6 7 | 23 | ok |
| **His/Gly, Suc** | 101.6 | 0.99 | 97.97 | 1.04 | 0.18 | - | 2.85 | 96.0 2 | 23 | ok |
| **PO₄**, **Tre** | 100.0 | 1.27 | 97.65 | 1.09 | 0.16 | 0.31 | 2.80 | 96.0 8 | 29 | ok |
| **PO₄**, **Suc** | 99.5 | 1.28 | 97.67 | 1.05 | 0.18 | 0.30 | 2.82 | 95.8 5 | 28 | ok |

The effects encountered (DLS size changes) are investigated below with various stabilizers (trehalose dihydrate and sucrose).

For this purpose, tests analogous to the above were carried out: it should be noted however that owing to the temporal discrepancy, some differences in the starting material are present or other/new analytical equipment has been used.

**Tab.7: Starting material, FTC (-20°C), with sucrose as stabilizer (DLS values were measured using the Zetasizer Nano-ZS from Malvern)**

| *All samples comprise: 25 mg*/*mL BAY 1213790, 0.05% (w*/*v) polysorbate 80, 5% (w*/*v) sucrose and are at pH 6* | | | |
|---|---|---|---|
| **Sample** | **Cloudiness [NTU]** | **DLS [nm]** | **Visually** |
| | **Starting material** | | |
| **5 mM histidine, 200 mM glycine** | 7.61 | 16.42 | OK |
| **10 mM histidine, 130 mM glycine** | 7.81 | 16.64 | OK |
| **30 mM histidine, 100 mM glycine** | 9.87 | 18.51 | OK |
| **50 mM histidine, 50 mM glycine** | 10.1 | 19.17 | OK |
| **50 mM Na₂HPO₄** | 10.1 | 20.24 | OK |

| | **After FTC (-20°C)** | | |
|---|---|---|---|
| **5 mM histidine, 200 mM glycine** | 7.45 | 16.41 | OK |
| **10 mM histidine, 130 mM glycine** | 7.47 | 16.23 | OK |
| **30 mM histidine, 100 mM glycine** | 10.5 | 18.35 | OK |
| **50 mM histidine, 50 mM glycine** | 9.72 | 19.22 | OK |
| **50 mM** | 10.5 | 19.57 | OK |

| **Sample** | **Cloudiness [NTU]** | **DLS [nm]** | **Visually** |
|---|---|---|---|
| **Na₂HPO₄** | | | |

**Tab.8: Starting material, FTC (-20°C), with trehalose dihydrate as stabilizer (DLS values were measured using the Zetasizer Nano-ZS from Malvern). All samples comprise: 25 mg/mL BAY 1213790, 0.05% (w/v) polysorbate 80, 5% (w/v) trehalose dihydrate and are at pH 6**

| **Sample** | **Cloudiness [NTU]** | **DLS [nm]** | **Visually** |
|---|---|---|---|
| | **Starting material** | | |
| **5 mM histidine, 200 mM glycine** | 7.28 | 16.49 | OK |
| **10 mM histidine, 130 mM glycine** | 9.44 | 17.45 | OK |
| **30 mM histidine, 100 mM glycine** | 8.62 | 18.39 | OK |
| **50 mM histidine, 50 mM glycine** | 8.98 | 18.97 | OK |
| **50 mM Na₂HPO₄** | 10.1 | 20.28 | OK |

| | **After FTC (-20°C)** | | |
|---|---|---|---|
| **5 mM histidine, 200 mM glycine** | 11.7 | 16.39 | OK |
| **10 mM histidine, 130 mM glycine** | 10.8 | 17.46 | OK |
| **30 mM histidine, 100** | 9.36 | 18.30 | OK |

| **Sample** | **Cloudiness [NTU]** | **DLS [nm]** | **Visually** |
|---|---|---|---|
| **mM glycine** | | | |
| **50 mM histidine, 50 mM glycine** | 10.5 | 19.00 | OK |
| **50 mM Na₂HPO₄** | 13.0 | 20.70 | OK |

### Viscosity

The viscosity of the formulation is concentration dependent and depending on the protein concentration is between 1.14 mPa^{∗}s (10 mg/mL BAY 1213790) and 2.25 mPa^{∗}s (40 mg/mL BAY 1213790). For the tests only the protein concentration was varied, while other constituents of the formulation were not modified (10/130 mM histidine/glycine at pH 6, 5% (w/v) trehalose dihydrate and 0.05% (w/v) polysorbate 80).

### Freeze-drying

25 mg/mL BAY 1213790 were formulated in a 10 mM histidine/130 mM glycine buffer (10/130 His/Gly) at pH 6 with 5% (w/v) trehalose dihydrate and 0.05% (w/v) polysorbate 80. The formulation was subsequently lyophilized with the freeze-drying cycle described in Table 9.

**Tab. 9: Freeze-drying cycle of the formulation (25 mg/mL BAY 1213790 in 10/130 His/Gly, 5% (w/v) trehalose dihydrate, 0.05% (w/v) polysorbate 80 at pH 6)**

| **Set surface temperature [°C]** | **Time [min]** | **Vacuum [µbar]** |
|---|---|---|
| **Freezing phase** | | |
| -5 | 30 | - |
| -5 | 60 | - |
| -45 | 40 | - |
| -45 | 210 | - |

| **Primary drying** | | |
|---|---|---|
| -45 | 60 | 100 |
| -10 | 60 | 100 |
| -10 | 3500 | 100 |

| **Secondary drying** | | |
|---|---|---|
| 40 | 60 | 40 |
| 40 | 720 | 40 |

After freeze-drying, the vials were stored at 2-8°C. Table 10 shows the stability data of the lyophilized formulation before and after storage for 36 months.

Further time points between 0 and 36 months were also measured and all data also here were within specification. The following data from Table 10 confirm that the selected formulation can be lyophilized and is subsequently stable even for a period of 3 years.

**Tab. 10: Stability data of the lyophilized formulation (25 mg/mL BAY 1213790 in 10/130 His/Gly, 5% (w/v) trehalose dihydrate, 0.05% (w/v) polysorbate 80 at pH 6)**

| **Test** | **Specification** | **Storage at 2-8°C** | |
|---|---|---|---|
| | | **t₀** | **t = 36 months** |
| **Formulation** | stable lyophilizate | conforms | conforms |
| **Colour** | White to whitish cake | conforms | conforms |
| **Clarity of the reconstituted solution** | Clear or not stronger opalescence than RS IV | <RSIII | <RSIII |
| **pH** | 5.5 - 6.5 | 6.1 | 6.1 |
| **Residual moisture** | max. 2.0% | 0.6 | 0.5 |
| **Visible particles** | Virtually free of visisble particles | conforms | conforms |
| **Particle size ≥ 25 µm (HIAC)** | max. 600 particles per vial | 3 | 3 |
| **Particle size ≥** | max. 6000 particles per vial | 62 | 64 |
| **10 µm (HIAC)** | | | |
| **IgG purity (CGE non-red., main peaks)** | Min. 90% | 99 | 99 |
| **IgG purity (CGE red., sum of HC+LC)** | Min. 95% | 98 | 99 |
| **Purity (SEC-HPLC), Monomer fraction** | Min. 93% | 98 | 96 |
| **Protein concentration (UV/VIS)** | 22.5 - 27.5 mg/mL | 25 | 24.7 |
| **Activity relative to reference (Bioassay)** | 60-140% | 104 | 101 |
| **Activity relative to reference (Biacore^{a}/ELISA ^{b})** | 50-150% | 108^{a} | 104^{b} |

### Conclusions:

Owing to the present results, the 10 mM histidine/130 mM glycine buffer system at pH 6.0 was selected which, with sufficient buffering capacity, has shown an optimal stabilizing effect during the stress tests.

Although the unstressed samples have similar stability features in different formulations, a clear difference was demonstrated in the stressed samples between the histidine-glycine system and the other formulations with reference to:
- Protein conformation (DLS)
- Intermolecular interactions (A2 value)
- Monomer fraction (SEC)
- Visible particles (visual image)

This shows that the histidine/glycine system at pH 6 has a better stabilizing effect on the BAY 1213790 antibody than other systems. This was not predictable by analysis of the unstressed samples.

## Claims

1. Liquid pharmaceutical formulation comprising anti-FXIa antibody BAY1213790 which consists of SEQ ID NO: 1 for the amino acid sequence for the heavy chain and SEQ ID NO: 2 for the amino acid sequence for the light chain at a concentration of 10 - 40 mg/ml, 10 mM histidine and 130mM glycine, wherein the formulation has a pH of 6.

2. Liquid pharmaceutical formulation according to Claim 1 comprising further ingredients selected from the group consisting of preservatives, carriers, wetting agents and stabilizers.

3. Liquid pharmaceutical formulation according to any of the preceding claims comprising 1-10% (w/v) of a stabilizer.

4. Liquid pharmaceutical formulation according to any of the preceding claims comprising 3-7% (w/v) trehalose dihydrate.

5. Liquid pharmaceutical formulation according to any of the preceding claims comprising wetting agents at a concentration of 0.001% to 0.5% (w/v).

6. Liquid pharmaceutical formulation according to Claim 5, wherein the wetting agent is polysorbate 80.

7. Liquid pharmaceutical formulation according to claim 1 comprising anti-FXIa antibody BAY1213790 at a concentration of 25 mg/ml, and further comprising 5% (w/v) trehalose dihydrate and 0.05% (w/v) polysorbate 80.

8. Lyophilizate obtainable by freeze-drying a liquid pharmaceutical formulation according to any of the preceding claims.

9. Lyophilizate according to Claim 8 comprising at most 2% residual water.

10. Dosage form comprising a liquid pharmaceutical formulation or a lyophilizate according to any of the preceding claims.

11. Dosage form according to Claim 10, wherein the dosage form is a syringe or an autoinj ector.

12. Liquid pharmaceutical formulation according to any of claims 1-7 for use in the treatment or prophylaxis of thrombotic or thromboembolic disorders.

## Patentansprüche

1. Pharmazeutische Flüssigformulierung, umfassend Anti-FXIa-Antikörper BAY1213790, der aus SEQ ID NO: 1 für die Aminosäuresequenz für die schwere Kette und SEQ ID NO: 2 für die Aminosäuresequenz für die leichte Kette besteht, in einer Konzentration von 10 - 40 mg/ml, 10 mM Histidin und 130 mM Glycin, wobei die Formulierung einen pH von 6 aufweist.

2. Pharmazeutische Flüssigformulierung nach Anspruch 1, umfassend weitere Inhaltsstoffe ausgewählt aus der Gruppe bestehend aus Konservierungsstoffen, Trägern, Netzmitteln und Stabilisatoren.

3. Pharmazeutische Flüssigformulierung nach einem der vorhergehenden Ansprüche, umfassend 1-10% (Gew./Vol.) eines Stabilisators.

4. Pharmazeutische Flüssigformulierung nach einem der vorhergehenden Ansprüche, umfassend 3-7% (Gew./Vol.) Trehalose-Dihydrat.

5. Pharmazeutische Flüssigformulierung nach einem der vorhergehenden Ansprüche, umfassend Netzmittel in einer Konzentration von 0,001% bis 0,5% (Gew./Vol.).

6. Pharmazeutische Flüssigformulierung nach Anspruch 5, wobei es sich bei dem Netzmittel um Polysorbat 80 handelt.

7. Pharmazeutische Flüssigformulierung nach Anspruch 1, umfassend Anti-FXIa-Antikörper BAY1213790 in einer Konzentration von 25 mg/ml und ferner umfassend 5% (Gew./Vol.) Trehalose-Dihydrat und 0,05% (Gew./Vol.) Polysorbat 80.

8. Lyophilisat, erhältlich über Gefriertrocknen einer pharmazeutischen Flüssigformulierung nach einem der vorhergehenden Ansprüche.

9. Lyophilisat nach Anspruch 8, umfassend höchstens 2% Restwasser.

10. Darreichungsform, umfassend eine pharmazeutische Flüssigformulierung oder ein Lyophilisat nach einem der vorhergehenden Ansprüche.

11. Darreichungsform nach Anspruch 10, wobei es sich bei der Darreichungsform um eine Spritze oder einen Autoinjektor handelt.

12. Pharmazeutische Flüssigformulierung nach einem der Ansprüche 1-7 zur Verwendung bei der Behandlung oder Prophylaxe thrombotischer oder thromboembolischer Störungen.

## Revendications

1. Formulation pharmaceutique liquide comprenant l'anticorps anti-FXIa BAY1213790, qui consiste en la SEQ ID NO: 1 pour la séquence d'acides aminés de la chaîne lourde et la SEQ ID NO: 2 pour la séquence d'acides aminés de la chaîne légère à une concentration de 10 à 40 mg/ml, 10 mM d'histidine et 130 mM de glycine, la formulation ayant un pH de 6.

2. Formulation pharmaceutique liquide selon la revendication 1 comprenant des ingrédients supplémentaires choisis dans le groupe consistant en les conservateurs, les véhicules, les agents mouillants et les stabilisants.

3. Formulation pharmaceutique liquide selon l'une quelconque des revendications précédentes comprenant 1 à 10 % (p/v) d'un stabilisant.

4. Formulation pharmaceutique liquide selon l'une quelconque des revendications précédentes comprenant 3 à 7 % (p/v) de tréhalose dihydraté.

5. Formulation pharmaceutique liquide selon l'une quelconque des revendications précédentes comprenant des agents mouillants à une concentration de 0,001 % à 0,5 % (p/v).

6. Formulation pharmaceutique liquide selon la revendication 5, dans laquelle l'agent mouillant est le polysorbate 80.

7. Formulation pharmaceutique liquide selon la revendication 1 comprenant l'anticorps anti-FXIa BAY1213790 à une concentration de 25 mg/ml, et comprenant en outre 5 % (p/v) de tréhalose dihydraté et 0,05 % (p/v) de polysorbate 80.

8. Lyophilisat pouvant être obtenu par lyophilisation d'une formulation pharmaceutique liquide selon l'une quelconque des revendications précédentes.

9. Lyophilisat selon la revendication 8 comprenant au plus 2 % d'eau résiduelle.

10. Forme posologique comprenant une formulation pharmaceutique liquide ou un lyophilisat selon l'une quelconque des revendications précédentes.

11. Forme posologique selon la revendication 10, la forme posologique étant une seringue ou un auto-injecteur.

12. Formulation pharmaceutique liquide selon l'une quelconque des revendications 1 à 7, destinée à être utilisée dans le traitement ou la prophylaxie de troubles thrombotiques ou thromboemboliques.
